# EUROPEAN PATENT APPLICATION

(11) **EP 4 700 012 A1**
(43) Date of publication of application: **25.02.2026**
(21) Application number: 24196143.2
(22) Date of filing: 23.08.2024
(51) Int. Cl.: C07C 209/26, C07C 209/84, C07C 211/14

(54) **PROCESS FOR THE PURIFICATION OF PENTAMETHYL DIETHYLENETRIAMINE**

(71) Applicant: BASF SE, 67056 Ludwigshafen am Rhein (DE)
(72) Inventor: Balkenhohl, Moritz, 67056 Ludwigshafen am Rhein (DE); Huber, Tatjana, 67056 Ludwigshafen am Rhein (DE); Risto, Eugen, 67056 Ludwigshafen am Rhein (DE); Pastre, Joerg, Dr., 67056 Ludwigshafen am Rhein (DE); Fuerstenberg, Sven, 67056 Ludwigshafen am Rhein (DE); Challand, Nina, Dr., 67056 Ludwigshafen am Rhein (DE)
(74) Representative: Schuck, Alexander

(57) **Abstract**

A process for the purification of pentamethyl diethylenetriamine (PMDETA) comprising one or more by-products selected from the group consisting of N,N,N",N"-tetramethyldiethylenetriamine, N,N,N'-trimethyl-N'-[2-(methylamino)ethyl]ethane-1,2-diamine and N,N,3-trimethyl-1-imidazolidineethanamine, comprising the steps:
a) providing a crude PMDETA mixture comprising one or more of the by-products;
b) adding a compound reactive with the by-products;
c) reacting the by-products with the reactive compound to obtain derivatives of the by-products;
d) distillative separation of PMDETA from the by-product derivatives obtained in step c) to obtain pure PMDETA.

## Description

The present invention concerns a process for the purification of pentamethyl diethylenetriamine (PMDETA) as well as a process for the preparation of PMDETA.

Pentamethyl diethylenetriamine or N,N,N',N',N"-pentamethyl diethylenetriamine (hereinafter referred to as "PMDETA") has the following structural formula:

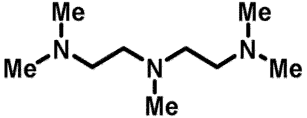

PMDETA is for instance used as a catalyst in polyurethane production as for instance taught in US5039713. It can be manufactured by the reductive amination of diethylenetriamine and formaldehyde, usually in a semi-batch mode or continuously.

A semi-batch mode is for instance described in US 5,105,013 (Dow). Example 1 thereof describes the production of PMDETA in an autoclave. The autoclave is charged with diethylenetriamine (DETA). Formaldehyde is added dropwise in an overall period of 15 hours. A 37 percent solution of formaldehyde in water, containing about 10 percent of methanol is used.

A semi-batch mode is also described in US 5,646,235 (Huntsman). Example 3 thereof describes the production of PMDETA in an autoclave. Formaldehyde is used as a methylformcel solution (55% formaldehyde/45% methanol).

The continuous production of PMDETA is for instance taught in DE 2137710 (BASF), DE 2545695 (BASF) and DE 2618580 (BASF).

DE 2137710 (BASF) explicitly teaches the manufacture of PMDETA in example 3. DETA and formaldehyde are continuously reacted in a molar ratio of about 5.6:1. Formaldehyde is employed as an aqueous solution. The reaction pressure amounts to 250 atm (i.e. about 253 bar). According to the specification a temperature of 40 to 200 °C, preferably 80 to 120 °C is applied. A molar ratio from stoichiometric ratio to a 60% molar excess of formaldehyde is taught, particularly a molar ratio of formaldehyde to N-H group(s) in the respective amine of 1 to 1.4.

DE 2545695 (BASF) explicitly teaches the manufacture of PMDETA in the example. DETA and formaldehyde are continuously reacted in a molar ratio of about 5:1. Formaldehyde is employed as an aqueous solution. The applied reaction pressure amounts to 200 bar. The reaction temperature is in the range of 80 to 105 °C.

DE 2618580 (BASF) explicitly teaches the manufacture of PMDETA in example 1. DETA and formaldehyde are continuously reacted in a molar ratio of about 5:1. Formaldehyde is employed as an aqueous solution. The applied reaction pressure amounts to 200 bar. The reaction temperature is in the range of 80 to 105 °C. The specification teaches a reaction pressure of 1 to 500 bar, preferably 100 to 200 bar, and a reaction temperature of 40 to 110 °C. According to the specification, the molar ratio of formaldehyde to N-H group(s) in the respective amine is in the range of 0.9:1 to 1.2:1.

PMDETA is obtained from DETA and formaldehyde as shown in the following reaction scheme:

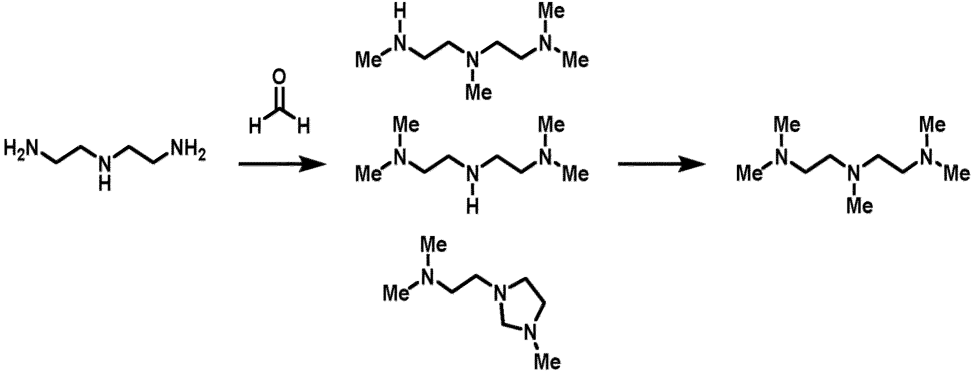

Because of its stoichiometry, the manufacture of PMDETA per se requires a large amount of formaldehyde (at least 5 equivalents of formaldehyde to theoretically obtain full DETA conversion). As intermediate products N,N,N',N"-tetramethyl diethylenetriamine and N,N,N",N"-tetramethyl diethylenetriamine occur. Another intermediate product being observed is N,N,3-trimethyl-1-imidazolidineethanamine (CAS = 143228-33-5, hereinafter referred to as "DMAEMI").

The intermediate products mentioned above have been identified in the crude reaction product as by-products. Particularly, the presence of DMAEMI constitutes a considerable problem because the separation by distillation of DMAEMI from PMDETA is, due to their similar boiling points, hardly possible.

It is therefore an object of the present invention to provide a process for the manufacture of pure pentamethyl diethylentriamine.

The object is achieved by a process for the purification of pentamethyl diethylentriamine (PMDETA) comprising one or more by-products selected from the group consisting of N,N,N",N"-tetramethyldiethylenetriamine, N,N,N'-trimethyl-N'-[2-(methylamino)ethyl]ethane-1,2-diamine and N,N,3-trimethyl-1-imidazolidineethanamine, comprising the steps:
a) providing a crude PMDETA mixture comprising one or more of the by-products;
b) adding a compound reactive with the by-products;
c) reacting the by-products with the reactive compound to obtain derivatives of the by-products;
d) distillative separation of PMDETA from the derivatives obtained in step c) to obtain pure PMDETA.

In general, the reactive compound comprises a C=O moiety. Reactive compounds to be added in step b) are preferably selected from aldehydes, ketones, carboxylic acids, and carboxylic acid derivatives.

More preferably, the reactive compound is selected from the group consisting of aldehydes and carboxylic acid derivatives, and is in particular selected from the group consisting of aldehydes, carboxylic acid anhydrides, carboxylic acid chlorides and carboxylic acid esters. In general, the aldehydes, ketones, carboxylic acids, and carboxylic acid derivatives contain exactly one corresponding functional group, selected from an aldehyde group, a keto group, a carboxylic acid group and a carboxylic acid derivative group, and no further functional group which is reactive with the by-products. I.e., the reactive compound contains either one aldehyde group, or one keto group, or one carboxylic acid group, or one carboxylic acid derivative group. The reactive compound can contain further functional groups which are not reactive with the by-products, such as an ether group.

In general, the reactive compound contains 1 to 10 carbon atoms, preferably 2 to 8 carbon atoms, more preferably 2 to 6 carbon atoms.

Particularly preferred reactive compounds are selected from the group consisting of methyl formiate, formyl morpholine, acetic anhydride, acetyl chloride and ethyl acetate. A very particularly preferred reactive compound is acetic anhydride.

The amount of the reactive compound added in step b) is generally in the range from 0.1 to 8 wt.-%, preferably from 0.2 to 4,0 wt.-%, based on total amount of the crude PM DETA mixture.

Reaction step c) is in general carried out at a reaction temperature in the range from 20 to 140°C, preferably from 40 to 120 °C. Step c) is in general carried out at a reaction pressure in the range from 0.25 to 5 bara, preferably from 0.5 to 3 bara.

In general, the reaction time in step c) is in the range from 0.4 to 120 min, preferably from 1 to 60 min. In a batch process, the reaction time is the residence time in the reactor. In a continuous process, the reaction time is the average residence time in the reactor.

The distillative separation in step d) is carried out at a distillation pressure in the range from 1 to 500 mbara, preferably from 5 to 200 mbara, more preferably from 50 to 100 mbara. The sump temperature should be kept below 140 °C in order to avoid reverse cleavage of the by-product derivative.

The distillative separation in step d) can be carried out as continuous or as a batch distillation.

The continuous distillative separation in step d) can be carried out in a two-column setup or a one-column setup. If acetic anhydride is used as the reactive compound, a PMDETA / acetic anhydride / acetic acid mixture can be separated as overhead stream of a first column and the by-product derivatives are obtained as bottom withdrawal stream. In a second distillation column pure PMDETA can be separated as bottom stream of the second distillation column, while PM DETA / acetic anhydride / acetic acid mixture is obtained as the overhead stream of the second distillation column. If a one-column setup is used, a PMDETA / acetic anhydride / acetic acid mixture can be separated as overhead stream, pure PMDETA can be separated as side withdrawal stream, and the by-product derivatives are obtained as bottom withdrawal stream of the single column.

The distillative separation in step d) can also be carried out as batch distillation. In such case, a first fraction contains the PMDETA / acetic anhydride / acetic acid mixture, a second fraction contains pure PMDETA. Remaining by-product derivatives remain in the sump oft he column.

The purity of PMDETA obtained in step d) is in general ≥ 99 % by weight, preferably ≥ 99.5 % by weight, in particular ≥ 99.7 % by weight.

The invention further relates to a process for the preparation of pentamethyl diethylenetriamine (PMDETA) comprising the steps:
(i) reacting diethylenetriamine (DETA) with formaldehyde to obtain a first crude PMDETA mixture containing by-products selected from the group consisting of N,N,N",N"-tetramethyldiethylenetriamine, N,N,N'-trimethyl-N'-[2-(methylamino)ethyl]ethane-1,2-diamine and N,N,3-trimethyl-1-imidazolidineethanamine, and low-boiling compounds;
(ii) distilling off the low boiling compounds from the first crude PMDETA mixture to obtain a second crude PMDETA mixture;
(iii) purifying the second crude PMDETA mixture in the process for the purification of PMDETA described above.

Low boiling compounds are compounds having a boiling point lower than that of PMDETA. Possible low boiling compounds are formaldehyde, methanol, water, and light-boiling amines.

In a preferred embodiment of the process of the invention, a methanolic formaldehyde solution is employed in step (i).

The term "methanolic formaldehyde solution" means that formaldehyde is employed in a solution that comprises methanol and preferably water. Preferably, the methanolic formaldehyde solution comprises formaldehyde in the range of 35 to 65 wt.-%, methanol in the range of 25 to 45 wt.-% and water in the range of 5 to 20 wt.-%.

The process of the invention can be conducted discontinuously (as a batch process), semi-continuously (as a semi-batch process) or continuously.

In certain embodiments of the inventive process, step (i) comprises continuously feeding DETA, a methanolic formaldehyde solution and hydrogen into one or more reactor(s) equipped with a heterogeneous hydrogenation catalyst and subjecting DETA and formaldehyde in such reactor(s) to reductive amination to obtain PMDETA.

In certain preferred embodiments of the inventive process, step (i) comprises continuously feeding DETA, a formaldehyde solution, preferably a methanolic formaldehyde solution, and hydrogen into one or more reactor(s) equipped with a heterogeneous hydrogenation catalyst and subjecting DETA and formaldehyde in such reactor(s) to reductive amination to obtain PMDETA, wherein the molar ratio of formaldehyde to DETA is in the range of 4.5:1 to < 5.9:1.

It has been found that DMAEMI formation can be reduced and longer run times without the occurrence of a selectivity drop realized when a molar ratio of DETA to formaldehyde below 5.9:1 is set.

The reductive amination can be conducted in one or more reactor(s). Preferred reactors are tube reactors. In case more than one reactor is employed, an interconnection in series or in parallel is possible. An example for a parallel interconnection is a tube bundle reactor. In principle, it is also possible to combine both kinds of interconnection, for example, by operating two or more tube bundle reactors in series.

The molar ratio of formaldehyde to DETA refers to the overall molar amount of formaldehyde and DETA being fed to the one or more reactor(s). It is possible to add formaldehyde and DETA at a reactor input and/or to add them at various inlets being distributed over the length of a reactor between its input and output. In case of an interconnection in series, formaldehyde and or DETA may also be added between two reactors. In case of more than one DETA feed and/or formaldehyde feed to the one or more reactor(s), the molar ratio is calculated from the sum over all molar feed rates of formaldehyde and the sum over all molar feed rates of DETA to the one or more reactor(s). Formaldehyde and DETA can be fed separately to the reactor(s), i.e. via separate inputs. It is also possible to mix them before feeding them into the reactor(s).

Usually, the reaction temperature is in the range of 30 to 300°C, preferably 30 to 250°C, more preferably 30 to 200°C. Increasing the lower limit of the temperature ranges results in reduced DMAEMI formation. Therefore, the reaction temperature is even more preferably 60 to 150°C, particularly preferably 80 to 130°C, more particularly preferably 90 to 130°C.

The reaction pressure is usually in the range of 50 to 300 bar, preferably 50 to 250 bar, more preferably 50 to 200 bar, even more preferably 60 bar to 150 bar, particularly preferably 80 to 150 bar, more particularly preferably 90 to 140 bar.

PMDETA obtained by the process according to the present invention may have a purity of ≥ 90 wt.-%, for instance a purity of 90 to 99 wt.-%. By-products comprised therein are N,N,N',N"-tetramethyl diethylenetriamine, N,N,N",N"-tetramethyl diethylenetriamine as well as DMAEMI.

In a preferred embodiment the process according to the invention is operated in a recycle gas mode. This means that low volatile components, in particular hydrogen, are separated from the obtained PMDETA and recycled to the reaction. The separation is usually carried out in a high-pressure separator into which the reactor effluent is fed. A high-pressure separator is a device (for instance a vessel) that is operated at a pressure slightly below or at the reaction pressure. The recycle gas comprises preferably at least 10% by volume, particularly from 50 to 100% by volume, very particularly from 80 to 100% by volume of Hz.

In a preferred embodiment the PMDETA is partially recycled to the reductive amination of DETA and formaldehyde. A recycle of PMDETA to the reaction helps to control the temperature profile in the one or more reactor(s). The more PMDETA is recycled, the lower is the rise of the temperature in the one or more reactor(s). Such recycle is usually affected after low volatile components (in particular hydrogen) have been separated from the PMDETA. Such separation can be carried out in a high-pressure separator as described above.

The weight ratio of the recycled PMDETA to the combined amount of methanolic formaldehyde solution and DETA being fed to the one or more reactors(s) can be in the range of 1:1 to 10:1, preferably 2:1 to 8:1. The weight ratio is calculated based on the mass flow rate of the respective streams comprising the PMDETA, DETA and methanolic formaldehyde solution.

The process according to the invention is conducted in the presence of a heterogeneous hydrogenation catalyst. The term heterogeneous catalyst designates a solid catalyst, preferably in form of particles, which is brought into contact with a liquid or gaseous medium, which is usually the reaction mixture comprising the starting materials and any intermediates and PMDETA already obtained.

Any heterogeneous catalyst that has sufficient hydrogenation activity can be used for the manufacture of PMDETA in accordance with the present invention. It may be a supported or an unsupported catalyst. An unsupported catalyst is preferred.

Preferably, the heterogeneous hydrogenation catalyst is devoid or substantially devoid of any palladium.

The heterogeneous hydrogenation catalyst is usually installed in the reactor, preferably as fixed bed.

The heterogeneous hydrogenation catalyst may comprise cobalt. Preferably it comprises cobalt and copper, more preferably cobalt, copper and manganese, even more preferably cobalt, copper, manganese and molybdenum, particularly preferably cobalt, copper, manganese, molybdenum, and phosphorus.

The heterogeneous hydrogenation catalyst can for instance be prepared by applying precipitation or impregnation methods. Suitable heterogeneous hydrogenation catalysts and respective methods for their production are for instance taught in EP 2043996 B1, WO 2011/067200 A1, WO 2011/067199 A1 and EP 2780109 B1 (all BASF).

In a very preferred embodiment, the heterogeneous hydrogenation catalyst comprises in the range of
5 to 90 wt.-%, preferably 10 to 60 wt.-%, cobalt,
1 to 40 wt.-%, preferably 2 to 30 wt.-%, copper,
0.1 to 30 wt.-%, preferably 1 to 10 wt.-%, manganese,
0.1 to 30 wt.-%, preferably 1 to 10 wt.-%, molybdenum, and
0.05 to 30 wt.-%, preferably 0.1 to 5 wt.-%, phosphorus,
based on the total weight of the heterogeneous hydrogenation catalyst.

In step (ii) of the process for the preparation of PMDETA, the low boiling compounds contained in the first crude PMDETA mixture are distilled off to obtain a second crude PMDETA mixture.

In case that a methanolic solution is employed in step (i), and in continuous operation, the distillation can be carried out in a two-column setup as follows: In a first distillation column, methanol is separated as overhead stream, and in a second distillation column, water is separated as overhead stream. The second crude PMDETA mixture is obtained as the bottom withdrawal stream of the second distillation column. If a one-column setup is used, methanol is separated as overhead stream, water is separated as side withdrawal stream, and the second crude PMDETA mixture is obtained as bottom withdrawal stream.

The distillative separation in step (ii) can also be carried out as batch distillation. In such case, a first fraction contains methanol, a second fraction contains water, the PMDETA mixture is obtained as third fraction overhead or as sump.

The invention is further illustrated by the following examples.

### Examples

### General

All raw materials and purified compounds are analyzed by GC-chromatography with the following parameters. GC column: RTX-5 Amin (30 m x 0.32 mm x 1.5 µm) 80°C-10°C/min-280°C/15min. Flow: 1.35 mL/min N₂.

The by-products are labelled as follows:

| | |
|---|---|
| BP1: | N,N,N",N"-tetramethyldiethylenetriamine (CAS = 40538-81-6) |
| BP2: | N,N,N'-trimethyl-N'-[2-(methylamino)ethyl]ethane-1,2-diamine (CAS = 61877-80-3) |
| BP3: | N,N,3-trimethyl-1-imidazolidineethanamine, "DMAEMI" (CAS = 143228-33-5) |

### Example 1-4: Derivatization of Byproducts 1-3 (BP1-BP3)

### Example 1:

PMDETA raw, containing BP1 (0.40%), BP2 (0.02%) and BP3 (0.61%) and PMDETA (97.4%), is submitted to a vessel and stirred at room temperature (20°C). Acetic anhydride (AczO, 0.4 w% of PMDETA raw) is added and the mixture stirred at room temperature for 5 min resulting in a derivatized reaction mixture containing BP1 (0.02%), BP2 (0.01%), BP3 (0.62%) and PMDETA (98.3).

### Example 2:

PMDETA raw, containing BP1 (0.98%), BP2 (0.33%) and BP3 (0.32%) and PMDETA (95.6%), is submitted to a vessel and stirred at room temperature. Acetic anhydride (AczO, 3.0 w% of PMDETA raw) is added and the mixture stirred at 60 °C for 1 h resulting in a derivatized reaction mixture containing BP1 (0.00%), BP2 (0.00%), BP3 (0.01%) and PMDETA (94.6%).

### Example 3:

PMDETA raw, containing BP1 (1.48%), BP2 (0.08%) and BP3 (0.37%) and PMDETA (95.3%), is submitted to a vessel and stirred at room temperature. Acetic anhydride (AczO, 3.0 w% of PMDETA raw) is added and the mixture stirred at 60 °C for 1 h resulting in a derivatized reaction mixture containing BP1 (0.00%), BP2 (0.00%), BP3 (0.00%) and PMDETA (95.1%).

### Example 4:

PMDETA raw, containing BP1 (1.51%), BP2 (0.30%) and BP3 (0.15%) and PMDETA (95.2%), is submitted to a vessel and stirred at room temperature. Acetic anhydride (AczO, 2.0 w% of PMDETA raw) is added and the mixture stirred at 60 °C for 1 h resulting in a derivatized reaction mixture containing BP1 (0.00%), BP2 (0.00%), BP3 (0.14%) and PMDETA (95.1%).

### Examples 5-8: PMDETA distillation

### Example 5

PMDETA derivatized from example 1 was submitted to distillation at 100 mbar with a distillation tower of 55-60 theoretical stages with sump temperatures ranging from 120-170 °C, head temperatures at 120-130 °C. PMDETA was isolated in a purity of 99.3% containing BP3 (0.6%).

### Example 6

PMDETA derivatized from example 2 was submitted to distillation at 100 mbar with a distillation tower of 55-60 theoretical stages with sump temperatures ranging from 130-185 °C and head temperatures at 120-130 °C. PMDETA was isolated in a purity of 99.9%. At sump temperatures of above 155 °C acetic acid (2 w%) was detected in the distillate.

### Example 7

PMDETA derivatized from example 3 was submitted to distillation at 100 mbar with a distillation tower of 55-60 theoretical stages with sump temperatures ranging from 130-180 °C and head temperatures at 100-130 °C. PMDETA was isolated in a purity of 99.9%. At sump temperatures of above 160 °C acetic acid (1 w%) was detected in the distillate.

### Example 8

PMDETA derivatized from example 4 was submitted to distillation at 100 mbar with a distillation tower of 25-30 theoretical stages with sump temperatures ranging from 130-180 °C and head temperatures at 110-130 °C. PMDETA was isolated in a purity of 99.7% containing BP3 (0.13%). At sump temperatures of above 150 °C acetic acid (4 w%) was detected in the distillate.

### Example 9: Continuous derivatization and distillation

PMDETA raw, containing BP1 (0.19%), BP2 (0.05%) and BP3 (1.20%) and PMDETA (95.4%), is continuously fed to a vessel at 80 °C and acetic anhydride (AczO, 4 w% of PMDETA raw feed) is fed continuously to this vessel. Average residence time in this vessel is 5 min. The derivatized reaction mixture contains BP1 (0.02%), BP2 (0.01%), BP3 (0.00%) and PMDETA (94.8%). Continuous distillation of this feed was performed at 40 mbar using a column with 44 theoretical stages. Sump temperatures ranged from 110-112 °C and head temperatures from 102-106 °C. PMDETA was isolated in a purity of 99.2% containing BP3 (0.30%).

## Claims

1. A process for the purification of pentamethyl diethylenetriamine (PMDETA) comprising one or more by-products selected from the group consisting of N,N,N",N"-tetramethyldiethylenetriamine, N,N,N'-trimethyl-N'-[2-(methylamino)ethyl]ethane-1,2-diamine and N,N,3-trimethyl-1-imidazolidineethanamine, comprising the steps:
a) providing a crude PMDETA mixture comprising one or more of the by-products;
b) adding a compound reactive with the by-products;
c) reacting the by-products with the reactive compound to obtain derivatives of the by-products;
d) distillative separation of PM DETA from the by-product derivatives obtained in step c) to obtain pure PMDETA.

2. The process according to claim 1, wherein the reactive compound comprises a C=O moiety.

3. The process according to claim 1 or 2, wherein the reactive compound is selected from the group consisting of aldehydes, ketones, carboxylic acids, and carboxylic acid derivatives.

4. The process according to claim 3, wherein the reactive compound is selected from the group consisting of aldehydes, carboxylic acid anhydrides, carboxylic acid chlorides and carboxylic acid esters.

5. The process according to any one of claims 1 to 4, wherein the reactive compound contains 1 to 10 carbon atoms, preferably 2 to 8 carbon atoms, more preferably 2 to 6 carbon atoms.

6. The process according any one of claims 1 to 5, wherein the reactive compound is selected from the group consisting of methyl formiate, formyl morpholine, acetic anhydride, acetyl chloride and ethyl acetate.

7. The process according to claim 6, wherein the reactive compound is acetic anhydride.

8. The process according to any one of claims 1 to 7, wherein the amount of the reactive compound added in step b) is in the range from 0.1 to 8 wt.-%, based on total amount of the crude PMDETA mixture.

9. The process according to any one of claims 1 to 8, wherein step c) is carried out at a reaction temperature in the range from 20 to 110°C.

10. The process according to any one of claims 1 to 9, wherein step c) is carried out at a reaction pressure in the range from 0.25 to 5 bara.

11. The process according to any one of claims 1 to 10, wherein the reaction time in step c) is in the range from 0.4 to 120 min.

12. The process according to any one of claims 1 to 11, wherein the distillative separation in step d) is carried out at a distillation pressure in the range from 1 to 500 mbara, preferably from 5 to 200 mbara, more preferably from 50 to 100 mbara.

13. A process for the preparation of pentamethyl diethylenetriamine (PMDETA) comprising the steps:
(i) reacting diethylenetriamine with formaldehyde to obtain a first crude PMDETA mixture containing by-products selected from the group consisting of N,N,N",N"-tetramethyldiethylenetriamine, N,N,N'-trimethyl-N'-[2-(methylamino)ethyl]ethane-1,2-diamine and N,N,3-trimethyl-1-imidazolidineethanamine, and low-boiling compounds;
(ii) distilling off the low boiling compounds to obtain a second crude PMDETA mixture;
(iii) purifying the second crude PMDETA mixture in a process for the purification of PMDETA according to any one of claims 1 to 12.

14. The process of claim 11, wherein a methanolic formaldehyde solution is employed in step (i).
